(19) Europäisches Patentamt
European Patent Office
Office européen des brevets

(11) Veröffentlichungsnummer: **0 100 874**
**B1**

(12) # EUROPÄISCHE PATENTSCHRIFT

(45) Veröffentlichungstag der Patentschrift: 07.03.90

(51) Int. Cl.$^5$: **C 07 J 5/00**, A 61 K 31/57, C 07 J 7/00// C07J21/00, C07J71/00

(21) Anmeldenummer: 83106649.3

(22) Anmeldetag: 07.07.83

(54) **Neue 6,16-Dimethylkortikoide, ihre Herstellung und Verwendung.**

(30) Priorität: 19.07.82 DE 3227312

(43) Veröffentlichungstag der Anmeldung: 22.02.84 Patentblatt 84/08

(45) Bekanntmachung des Hinweises auf die Patenterteilung: 07.03.90 Patentblatt 90/10

(84) Benannte Vertragsstaaten: AT BE CH DE FR GB IT LI LU NL SE

(56) Entgegenhaltungen:
EP-A- 0 054 786
BG-A- 898 291
DE-B- 1 902 340
GB-A- 898 292
GB-A- 931 221
GB-A- 1 047 519
GB-A- 1 544 642
US-A- 3 054 811
US-A- 4 154 748
SYNTHESIS 1982, Nr.1, Jan. 1982, Georg Thieme Verlag, Stuttgart-New York, US KLAUS ANNEN et al.: "A Simple Method for 6-Methylenation of 3-Oxo-Delta 4 -steroids", Seiten 34-40.

Die Akte enthält technische Angaben, die nach dem Eingang der Anmeldung eingereicht wurden und die nicht in dieser Patentschrift enthalten sind.

(73) Patentinhaber: SCHERING AKTIENGESELLSCHAFT
Berlin und Bergkamen
Müllerstrasse 170/178 Postfach 65 03 11
D-1000 Berlin 65 (DE)

(72) Erfinder: Annen, Klaus, Dr.
Seegefelderstrasse 194
D-1000 Berlin 20 (DE)
Erfinder: Laurent, Henry, Dr.
Glambecker Weg 21
D-1000 Berlin 28 (DE)
Erfinder: Hofmeister, Helmut, Dr.
Weislingenstrasse 4
D-1000 Berlin 28 (DE)
Erfinder: Wiechert, Rudolf, Prof. Dr.
Petzower Strasse 8a
D-1000 Berlin 39 (DE)
Erfinder: Töpert, Michael, Dr.
Sigismundkorso 58
D-1000 Berlin 28 (DE)
Erfinder: Wendt, Hans, Dr.
Ernst-Ring-Strasse 14
D-1000 Berlin 38 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 100 874 B1

Jouve, 18, rue St-Denis, 75001 Paris, France

**Beschreibung**

Die Erfindung betrifft neue 6,16-Dimethylkortikoide der allgemeinen Formel I

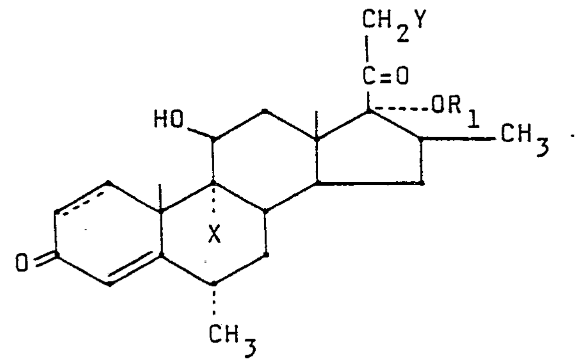

(I)

worin
die Bindung ⋯ eine Einfachbindung oder eine Doppelbindung darstellt und
X ein Wasserstoffatom, ein Fluoratom, oder ein Chloratom
$R_1$ eine Formylgruppe, eine 2 bis 8 Kohlenstoffatome enthaltende Alkanoylgruppe oder Alkoxyalkylgruppe oder eine Benzoylgruppe und
Y ein Chloratom, eine Hydroxygruppe, eine Formylgruppe, eine 2 bis 8 Kohlenstoffatome enthaltende Alkanoyloxygruppe oder eine Benzoyloxygruppe bedeuten.

Ferner betrifft die Erfindung ein Verfahren zur Herstellung von 6,16-Dimethylkortikoiden der allgemeinen Formel I, welches dadurch gekennzeichnet ist, daß man in an sich bekannter Weise

a) den Epoxidring eines Steroides der allgemeinen Formel II

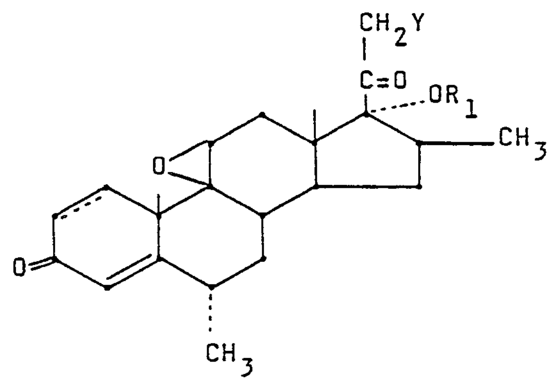

(II)

worin ⋯ $R_1$ und Y die im Patentanspruch 1 genannte Bedeutung besitzen, mit Fluorwasserstoff öffnet oder

b) an die $\Delta^{9(11)}$-Doppelbindung eines Steroids der allgemeinen Formel III

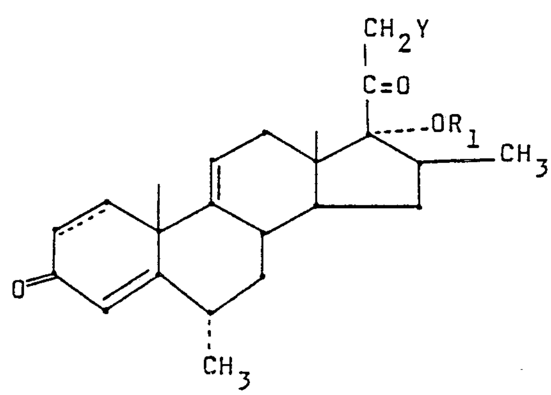

(III)

worin ⋯ $R_1$ und Y die im Patentanspruch 1 genannte Bedeutung besitzen unterchlorige oder

unterbromige Säure anlagert und gewünschtenfalls aus den 9-Chlor- oder 9-Bromsteroiden der allgemeinen Formel I das in der 9-Position ständige Halogenatom eliminiert, 21-Acetoxysteroide der allgemeinen Formel I verseift oder 21-Hydroxysteroide der allgemeinen Formel I verestert.

Letztlich betrifft die Erfindung Steroide der allgemeinen Formel VI

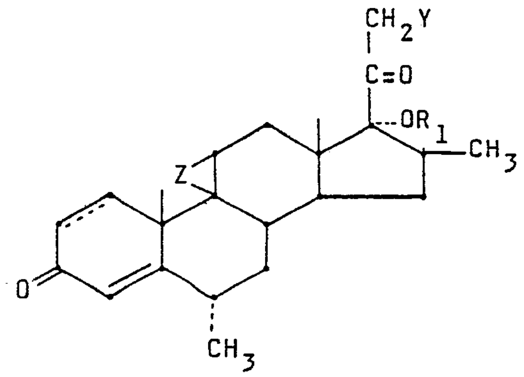

(VI)

worin ⋯ $R_1$ und Y die im Patentanspruch 1 genannte Bedeutung besitzen und

Z eine Kohlenstoff-Kohlenstoff-Bindung oder ein Sauerstoffatom bedeutet, welche als Ausgangssubstanzen für das erfindungsgemäße Verfahren Anwendung finden.

Die neuen 6,16-Dimethylkortikoide können als Substituenten $R_1$ eine Formylgruppe, eine 2 bis 8 (vorzugsweise 2 bis 6) Kohlenstoffatome enthaltende Alkanoylgruppe oder Alkoxyalkylgruppe oder eine Benzoylgruppe enthalten. Geeignete Alkanoylgruppen $R_1$ sind beispielsweise die Acetylgruppe, Proionylgruppe, die Butyrylgruppe, die Isobutyrylgruppe, die Valerianylgruppe, die 3-Methylbutyrylgruppe, die Trimethylacetylgruppe oder die Hexanoylgruppen. Als Alkoxyalkylgruppen $R_2$ seien insbesondere Alkoxymethylgruppen, wie die Methoxymethylgruppe, die Ethoxymethylgruppe, die Propyloxymethylgruppe, die Isopropyloxymethylgruppe, die Butyloxymethylgruppe, die Isobutyloxymethylgruppe oder die tert.-Butyloxymethylgruppe beispielsmäßig genannt.

Als Substituenten Y können die 6,16-Dimethylkortikoide ein Chloratom, eine Hydroxygruppe, eine 2 bis 8 (vorzugsweise 2 bis 6) Kohlenstoffatome enthaltende Alkanoyloxygruppe oder eine Benzoyloxygruppe enthalten. Geeignete Alkanoyloxygruppen sind beispielsweise solche, die sich von den obengenannten Alkanoylgruppen ableiten.

Die 6,16-Dimethylkortikoide der allgemeinen Formel I zeichnen sich bei topischer Applikation durch eine ausgezeichnete antiinflammatorische Wirksamkeit aus. Darüberhinaus zeichnen sie sich durch eine ausgezeichnete Dissoziation zwischen erwünschter topischer Wirksamkeit welche noch stärker ausgeprägt ist, als diejenige des Betamethasondipropionates und des Beclomethasondipropionats (GB-A 1047 519 und GB-A 1544 642). Die in der GB-A 898.291 beschriebenen Kortikoide gehören nicht zu der Klasse der topisch hochwirksamen Substanzen, da sie eine freie 17α-Hydroxygruppe besitzen.

Die neuen 6,16-Dimethylkortikoide der allgemeinen Formel I eignen sich in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel : Lösungen, Lotionen, Salben, Cremen oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Kortikoide auch in Form von Kapseln, Tabletten oder Dragees, die vorzugsweise 10 bis 200 mg Wirkstoff enthalten und oral appliziert werden oder in Form von Suspensionen, die vorzugsweise 100 bis 500 mg Wirkstoff pro Dosiseinheit enthalten und rektal appliziert werden. Auch zur Behandlung allergischer Erkrankungen des Darmtraktes, wie der Kolitis ulcerosa und der Kolitis granulomatosa.

Die neuen 6,16-Dimethylkortikoide können unter den Bedingungen hergestellt werden, wie sie in den deutschen Patentanmeldungen Nr. P 26 45 105.8 und 28 03 661.5 beschrieben sind.

Die nachfolgenden Ausführungsbeispiele dienen zur Erläuterung des erfindungsgemäßen Verfahrens.

Beispiel 1

A) Aus einer Lösung von 24.0 g 17α.21-Dihydroxy-16β-methyl-4.9(11)-pregnadien-3.20-dion und 2.4 g Pyridiniumtosylat in 180 ml Dimethylformamid und 1.7 l Benzol werden bei 130 °C über einen Wasserabscheider 720 ml Benzol abdestilliert. In die heiße Reaktionslösung läßt man 58 ml Orthopropionsäuretriethylester langsam zulaufen und destilliert anschließend weiter Benzol und andere leichtflüchtige Reaktionskomponenten ab. Man fügt nun 29 ml Pyridin hinzu und engt im Vakuum zur Trockne ein. Es wird 17α.21-(1-Ethoxy-propyliden-dioxy)-16β-methyl-4.9(11)-pregnadien-3.20-dion als Öl isoliert.

B) Das rohe 17α.21-(1-Ethoxy-propylidendioxy)-16β-methyl-4.9(11)-pregnadien-3.20-dion wird in 720 ml Methanol gelöst und mit einem Gemisch aus 258 ml O.1N wäßriger Essigsäure und 28.8 ml 0.1M wäßriger Natriumacetat-Lösung 1.5 h bei 100 °C Badtemperatur gerührt. Man engt die Lösung auf 1/3 ihres Volumens ein, gibt auf Wasser und wäscht die Essigesterextrakte neutral. Nach dem Trocknen und Einengen wird das Rohprodukt an 2 kg Kieselgel mit einem Methylenchlorid-Aceton-Gradienten (0-15 % Aceton) gereinigt. Man erhält 23.7 g 21-Hydroxy-16β-methyl-17α-propionyloxy-4.9(11)-pregnadien-3.20-dion vom Schmp. 192 °C.

C) 7.9 g 21-Hydroxy-16β-methyl-17α-propionyloxy-4.9(11)-pregnadien-3.20-dion werden in 79 ml Pyridin und 39 ml Acetanhydrid 1 h bei Raumtemperatur gerührt. Nach Eiswasserfällung und üblicher Aufarbeitung isoliert man 8.15 g 21-Acetoxy-16β-methyl-17α-propionyloxy-4.9(11)-pregnadien-3.20-dion vom Schmp. 95-96 °C.

D) Eine Suspension von 10.0 g Natriumacetat in 300 ml Chloroform und 300 ml Formaldehyddimethylacetal wird mit 19 ml Phosphoroxychlorid 1 h bei 65 °C Badtemperatur gerührt. Nach Zugabe von 10.0 g 21-Acetoxy-16β-methyl-17α-propionyloxy-4.9(11)-pregnadien-3.20-dion tropft man weitere 19 ml Phosphoroxychlorid hinzu und rührt 6.5 h bei 65 °C weiter. Die abgekühlte Reaktionslösung wird mit soviel einer gesättigten Sodalösung behandelt, bis die wäßrige Phase alkalisch bleibt. Man trennt die organische Phase ab, wäscht neutral und engt nach dem Trocknen ein. Das Rohprodukt wird an 700 g Kieselgel mit einem Hexan-Essigester-Gradienten (0-50 % Essigester) gereinigt. Man isoliert 7.5 g 21-Acetoxy-16β-methyl-6-methylen-17α-propionyloxy-4.9(11)-pregnadien-3.20-dion vom Schmp. 206-207 °C.

E) 7.0 g 21-Acetoxy-16β-methyl-6-methylen-17α-propionyloxy-4.9(11)-pregnadien-3.20-dion und 1.4 g Palladium/Aktivkohle (9.64 %ig) werden in 350 ml Isopropanol suspendiert und mit 21 ml Cyclohexen 5 h bei 120 °C Badtemperatur gerührt. Nach dem Abkühlen wird der Katalysator abgesaugt, mit Methylenchlorid gewaschen und die vereinigten Filtrate mit 40 ml konz. Salzsäure 0.5 h bei Raumtemperatur gerührt. Man engt die Reaktionslösung auf 1/3 ihres Volumens ein, gibt auf Eiswasser und arbeitet wie üblich auf. Das Rohprodukt wird an 600 g Kieselgel mit einem Hexan-Essigester-Gradienten (0-50 % Essigester) gereinigt. Ausbeute 5.4 g 21-Acetoxy-6α.16β-dimethyl-17α-propionyloxy-4.9(11)-pregnadien-3.20-dion. Schmp. 184-185 °C.

F) Eine Suspension von 4.4 g 21-Acetoxy-6α.16β-dimethyl-17α-propionyloxy-4.9(11)-pregnadien-3.20-dion in 68 ml Dioxan und 4.4 ml Wasser werden bei einer Innentemperatur von 20 °C portionsweise mit 3.3 g N-Bromsuccinimid versetzt. Beim Zutropfen einer Lösung von 0.35 ml 70 %iger Perchlorsäure in 5.3 ml Wasser darf die Innentemperatur 23 °C nicht überschreiten. Man rührt 45 min bei 20 °C Innentemperatur weiter, kühlt auf + 15 °C herunter und neutralisiert tropfenweise mit einer Lösung aus 1.6 g Natriumacetat und 1.0 g Natriumsulfit in 9.7 ml Wasser. Dabei darf die Innentemperatur 23 °C nicht übersteigen. Nach Zugabe von 60 ml Methanol wird das Reaktionsgemisch 15 min bei Raumtemperatur und nach Zugabe von 172 ml Wasser 3 h 0 °C weitergerührt. Schließlich saugt man den Niederschlag ab, wäscht den Rückstand mit Wasser neutral und trocknet bei 70 °C im Vakuumtrockenschrank. Das Rohprodukt wird an 350.0 g Kieselgel mit einem methylenchlorid-Aceton-Gradienten (0-8 % Aceton) gereinigt. Man erhält 3.2 g 21-Acetoxy-9α-brom-11β-hydroxy-6α.16β-dimethyl-17α-propionyloxy-4-pregnen-3.20-dion. Schmp. 176-178 °C.

G) 600 mg 21-Acetoxy-9α-brom-11β-hydroxy-6α.16β-dimethyl-17α-propionyloxy-4-pregnen-3.20-dion werden in 12.5 ml wasserfreiem Tetrahydrofuran suspendiert und nach Zugabe von 0.63 ml Tributylzinnhydrid und 20 mg Azobisisobutyronitril 0.5 h refluxiert. Man engt im Wakuum ein und reinigt den Rückstand an 100 g Kieselgel mit einem Hexan-Essigester-Gradienten (0-50 % Essigester). Ausbeute 320 mg 21-Acetoxy-11β-hydroxy-6α.16β-dimethyl-17α-propionyloxy-4-pregnen-3.20-dion. Schmp. 139-140 °C.

Beispiel 2

A) 34.4 g 21-Hydroxy-16β-methyl-17α-propionyloxy-4.9(11)-pregnadien-3.20-dion werden analog Beispiel 1 C) mit Propionsäureanhydrid umgesetzt und aufgearbeitet. Rohausbeute 40.0 g 16β-Methyl-17α.21-dipropionyloxy-4.9(11)-pregnadien-3.20-dion. Schmp. 95-97 °C.

B) 22.0 g 16β-Methyl-17β.21-dipropionyloxy-4.9(11)-pregnadien-3.20-dion werden unter den Bedingungen des Beispiels 1 D) mit Formaldehyddimethylacetal und Phosphoroxychlorid umgesetzt, aufgearbeitet und gereinigt. Man isoliert 13.5 g 16β-Methyl-6-methylen-17α.21-dipropionyloxy-4.9(11)-pregnadien-3.20-dion mit dem Schmp. 169-171 °C.

C) Unter den Bedingungen des Beispiels 1 E) werden 13.0 g 16β-Methyl-6-methylen-17α.21-dipropionyloxy-4.9(11)-pregnadien-3.20-dion mit Palladium/A-Kohle und Cyclohexen hydriert, aufgearbeitet und gereinigt. Ausbeute 9.8 g 6α.16β-Dimethyl-17α.21-dipropionyloxy-4.9(11)-pregnadien-3.20-dion. Schmp. 170-172 °C.

D) 4.0 g 6α.16β-Dimethyl-17α.21-dipropionyloxy-4.9(11)-pregnadien-3.20-dion werden analog Beispiel 1 F) mit N-Bromsuccinimid und Perchlorsäure umgesetzt, aufgearbeitet und gereinigt. Man erhält 2.9 g 9β-Brom-11β-hydroxy-6α.16β-dimethyl-17α.21-dipropionyloxy-4-pregnen-3.20-dion. Schmp. 177-178 °C.

E) Wie im Beispiel 1 G) beschrieben, wird 1.0 g 9α-Brom-11β-hydroxy-6α.16β-dimethyl-17α.21-dipropionyloxy-4-pregnen-3.20-dion mit Tributylzinnhydrid debromiert, aufgearbeitet und gereinigt. Ausbeute 710 mg 11β-Hydroxy-6α.16β-dimethyl-17α.21-dipropionyloxy-4-pregnen-3.20-dion. Schmp. 132-134 °C.

Beispiel 3

A) Eine Lösung von 1.0 g 17α.21-(1-Ethoxy-propylidendioxy)-16β-methyl-4.9(11)-pregnadien-3.20-dion in 50 ml Dimethylformamid wird nach tropfenweiser Zugabe von 1 ml Trimethylchlorsilan 23 h bei 80 °C Badtemperatur gerührt. Nach der Eiswasserfällung und üblichen Aufarbeitung reinigt man das Rohprodukt an 150 g Kieselgel mit einem Methylenchlorid-Aceton-Gradienten (0-8 % Aceton). Ausbeute 680 mg 21-Chlor-16β-methyl-17α- propionyloxy-4.9(11)-pregnadien-3.20-dion. Schmp. 197-199 °C.

B) 15.3 g 21-Chlor-16β-methyl-17α-propionyloxy-4.9(11)-pregnadien-3.20-dion werden analog Beispiel 1 D) mit Formaldehyd-dimethylacetal und Phosphoroxychlorid umgesetzt, aufgearbeitet und gereinigt. Man isoliert 9.6 g 21-Chlor-16β-methyl-6-methylen-17α-propionyloxy-4.9(11)-pregnadien-3.20-dion. Schmp. 199-200 °C.

C) Unter den Bedingungen des Beispiels 1 E) werden 7.2 g 21-Chlor-16β-methyl-6-methylen-17α-propionyloxy-4.9(11)-pregnadien-3.20-dion hydriert, aufgearbeitet und gereinigt. Es werden 5.4 g 21-Chlor-6α.16β-dimethyl-17α-propionyloxy-4.9(11)-pregnadien-3.20-dion isoliert. Schmp. 155-157 °C.

D) Wie im Beispiel 1 F) beschrieben, werden 5.0 g 21-Chlor-6α.16β-dimethyl-17α-propionyloxy-4.9(11)-pregnadien-3.20-dion mit N-Bromsuccinimid und Perchlorsäure umgesetzt, aufgearbeit und chromatographiert. Ausbeute 5.3 g 9α-Brom-21-chlor-11β-hydroxy-6α.16β-dimethyl-17α-propionyloxy-4-pregnen-3.20-dion. Schmp. 185-187 °C.

E) 2.0 g 9α-Brom-21-chlor-11β-hydroxy-6α.16β-dimethyl-17α-propionyloxy-4-pregnen-3.20-dion werden analog Beispiel 1 G) debromiert, aufgearbeitet und chromatographiert. Man isoliert 751 mg 21-Chlor-11β-hydroxy-6α.16β-dimethyl-17α-propionyloxy-4-pregnen-3.20-dion. Schmp. 219-221 °C.

Beispiel 4

A) Eine Lösung von 9.5 g 6α-16β-Dimethyl-17α.21-dipropionyloxy-4.9(11)-pregnadien-3.20-dion in 475 ml Dioxan wird mit 9.5 g Dichlorodicyanobenzochinon 15 h refluxiert. Nach dem Abkühlen und Abfiltrieren engt man das Filtrat zur Trockne ein. Das Rohprodukt wird an 750 g Kieselgel mit einem Hexan-Essigester-Gradienten (0-50 % Essigester) gereinigt. Rohausbeute 5.73 g 6α.16β-Dimethyl-17α.21-dipropionyloxy-1.4.9(11)-pregnatrien-3.20-dion, die in 20 ml siedendem Ethanol tropfenweise mit einer Lösung von 5.73 g $Na_2S_2O_5$ in 8 ml Wasser behandelt werden. Nach 2 h wird das Reaktionsgemisch in der Weise destilliert, daß das Volumen in der Destillationsblase durch Wasserzufuhr erhalten bleibt und das Brückenthermometer 99 °C anzeigt. Man kühlt die Destillationsblase auf + 20 °C ab, filtriert ab, wäscht den Rückstand gründlich mit Wasser und löst ihn in Methylenchlorid. Die organische Lösung wird nach dem Trocknen über eine Kieselgelschicht filtriert und anschließend eingeengt. Ausbeute 4.7 g. Schmp. 188-190 °C.

B) 3.0 g 6α.16β-Dimethyl-17α-21-dipropionyloxy-1.4.9(11)-pregnatrien-3.20-dion werden in 30 ml Dioxan gelöst und nach Zugabe von 2.8 g N-Bromsuccinimid tropfenweise mit 15 ml einer 10 %igen Perchlorsäure versetzt. Man rührt 0.5 h bei Raumtemperatur, gibt auf Eiswasser und arbeitet wie üblich auf. Man isoliert 3.5 g 9α-Brom-11β-hydroxy-6α.16β-dimethyl-17α.21-dipropionyloxy-1.4-pregnadien-3.20-dion. Schmp. 186-187 °C.

C) Unter den Bedingungen des Beispiels 1 G) wird 1.0 g 9α-Brom-11β-hydroxy-6α.16β-dimethyl-17α.21-dipropionyloxy-1,4-pregnadien-3.20-dion mit Tributylzinnhydrid debromiert, aufgearbeitet und gereinigt. Man erhält 760 mg 11β-Hydroxy-6α.16β-dimethyl-17α.21-dipropionyloxy-1,4-pregnadien-3.20-dion. Schmp. 130-132 °C.

Beispiel 5

A) 5.2 g 21-Chlor-6α.16β-dimethyl-17α-propionyloxy-4.9(11)-pregnadien-3.20-dion werden unter den Bedingungen des Beispiels 4 A) dehydriert, aufgearbeitet und gereinigt. Ausbeute 3.2 g 21-Chlor-6α.16β-dimethyl-17α-propionyloxy-1.4.9(11)-pregnatrien-3.20-dion. Schmp. 199-200 °C.

B) Wie im Beispiel 4 B) beschrieben, setzt man 2.2 g 21-Chlor-6α.16β-dimethyl-17α-propionyloxy-1.4.9(11)pregnatrien-3.20-dion mit 2.0 g N-Bromsuccinimid um, arbeitet auf und isoliert 2.5 g 9α-Brom-21-chlor-11β-hydroxy-6α.16β-dimethyl-17α-propionyloxy-1,4-pregnadien-3.20-dion. Schmp. 180-182 °C.

C) 700 mg 9α-Brom-21-chlor-11β-hydroxy-6α.16β-dimethyl-1,4-pregnadien-3.20-dion werden analog Beispiel 4 C) debromiert, aufgearbeitet und chromatographiert. Es werden 520 mg 21-Chlor-11β-hydroxy-6α.16β-dimethyl-17α-propionyloxy-1.4-pregnadien-3.20-dion isoliert. Schmp. 205-206 °C.

Beispiel 6

A) Analog Beispiel 1 A) wird 1.0 g 11β.17α.21-Trihydroxy-6α.16β-dimethyl-4-pregnen-3.20-dion mit 1.2 ml Orthobuttersäuretriethylester umgesetzt und aufgearbeitet. Man isoliert 17α.21-(1-Ethoxybutylidendioxy)-11β-hydroxy-6α.16β-dimethyl-4-pregnen-3.20-dion als Öl.

B) Das rohe 17α.21-(1-Ethoxybutylidendioxy)-11β-hydroxy-6α.16β-dimethyl-4-pregnen-3.20-dion wird unter den Bedingungen des Beispiels 1 B) hydrolysiert und aufgearbeitet. Das Rohprodukt wird an 100 g Kieselgel mit einem Methylenchlorid-Aceton-Gradienten (0-20 % Aceton) gereinigt. Ausbeute 810 mg 17α-Butyryloxy-11β.21-dihydroxy-6α-16β-dimethyl-4-pregnen-3.20-dion.

Beispiel 7

A) 1.0 g 11β.17α.21-Trihydroxy-6α.16β-dimethyl-1.4-pregnadien-3.20-dion wird unter den Bedingungen des Beispiels 1 A) mit Orthobenzoesäuretriethylester umgesetzt und aufgearbeitet. Dabei wird das 17α.21-(1-Ethoxybenzylidendioxy)-11β-hydroxy-6α.16β-dimethyl-1.4-pregnadien-3.20-dion als Öl isoliert.

B) Analog Beispiel 1 B) wird das rohe 17α.21-(1-Ethoxybenzylidendioxy)-11β-hydroxy-6α.16β-dimethyl-1.4-pregnadien-3.20-dion hydrolysiert und aufgearbeitet. Das Rohprodukt wird an 100 g Kieselgel mit einem Methylenchlorid-Aceton-Gradienten (0-8 % Aceton) gereinigt. Man isoliert 680 mg 17α-Benzoyloxy-11β.21-dihydroxy-6α.16β-dimethyl-1.4-pregnadien-3.20-dion.

Beispiel 8

A) Unter den Bedingungen des Beispiels 1 A) wird 1.0 g 11β.17α.21-Trihydroxy-6α.16β-dimethyl-4-pregnen-3.20-dion mit Orthoessigsäuretriethylester umgesetzt und aufgearbeitet. Man erhält das 17α.21-(1-Ethoxy-ethylidendioxy)-11β-hydroxy-6α.16β-dimethyl-4-pregnen-3.20-dion als Öl.

B) Das rohe 17α.21-(1-Ethoxy-ethylendioxy)-11β-hydroxy-6α.16β-dimethyl-4-pregnen-3.20-dion wird, wie im Beispiel 1 B) beschrieben, hydrolysiert und aufgearbeitet. Das Rohprodukt wird an 100 g Kieselgel mit einem Methylenchlorid-Aceton-Gradienten (0-15 % Aceton) gereinigt. Ausbeute 840 mg 17α-Acetoxy-11β.21-dihydroxy-6α.16β-dimethyl-4-pregnen-3.20-dion.

Beispiel 9

A) 3.0 g 21-Acetoxy-17α-hydroxy-16β-methyl-4.9(11)-pregnadien-3.20-dion werden analog Beispiel 1 D), jedoch mit Formaldehyddiethylacetal, umgesetzt. Nach einer Reaktionszeit von 1 h wird aufgearbeitet und gereinigt. Ausbeute 1.5 g 21-Acetoxy-17α-hydroxy-16β-methyl-6-methylen-4.9(11)-pregnadien-3.20-dion. Schmp. 122-123 °C.

B) Analog Beispiel 1 E) werden 1.4 g 21-Acetoxy-17α-hydroxy-16β-methyl-6-methylen-4.9(11)-pregnadien-3.20-dion mit Cyclohexen und Palladium/A-Kohle hydriert, aufgearbeitet und gereinigt. Man erhält 830 mg 21-Acetoxy-17α-hydroxy-6α. 16β-dimethyl-4.9(11)-pregnadien-3.20-dion. Schmp. 197-199 °C.

C) 5.0 g 21-Acetoxy-17α-hydroxy-6α. 16β-dimethyl-4.9(11)-pregnadien-3.20-dion werden analog

Beispiel 4 A) dehydriert, aufgearbeitet und gereinigt. Ausbeute 3.5 g 21-Acetoxy-17α-hydroxy-6α. 16β-dimethyl-1.4.9(11)-pregnatrien-3.20-dion. Schmp. 215-216 °C.

D) 3.5 g 21-Acetoxy-17α-hydroxy-6α. 16β-dimethyl-1.4.9(11)-pregnatrien-3.20-dion werden in 25 ml wasserfreiem Methylenchlorid und 16 ml Formaldehyddimethylacetal gelöst und portionsweise mit einem Gemisch aus 5.0 g Kieselgur W20 und 2.5 g Phosphorpentoxid versetzt. Man rührt 45 min bei Raumtemperatur nach, saugt ab und eluiert den Rückstand mehrmals mit Methylenchlorid, das 3-5 % Triethylamin enthält. Das Rohprodukt wird an 500 g Kieselgel mit einem Methylenchlorid-Aceton-Gradienten (0-10 % Aceton) gereinigt. Man isoliert 2.8 g 21-Acetoxy-17α-methoxymethoxy-6α. 16β-dimethyl-1.4.9-pregnatrien-3.20-dion.

E) Unter den Bedingungen des Beispiels 4 B) werden 2.8 g 21-Acetoxy-17α-methoxymethoxy-6α. 16β-dimethyl-1.4.9-pregnatrien-3.20-dion mit N-Bromsuccinimid umgesetzt, aufgearbeitet und gereinigt. Ausbeute 2.7 g 21-Acetoxy-9α-brom-11β-hydroxy-17α-methoxymethoxy-6α. 16β-dimethyl-1.4-pregnadien-3.20-dion.

F) Analog Beispiel 1 G) werden 2.6 g 21-Acetoxy-9α-brom-11β-hydroxy-17α-methoxymethoxy-6α. 16β-dimethyl-1.4-pregnadien-3.20-dion mit Tributylzinnhydrid debromiert, aufgearbeitet und gereinigt. Man isoliert 2.0 g 21-Acetoxy-11β-hydroxy-17α-methoxymethoxy-6α. 16β-dimethyl-1.4-pregnadien-3.20-dion.

## Beispiel 10

Eine Lösung von 700 mg 21-Acetoxy-11β-hydroxy-17α-methoxymethoxy-6α. 16β-dimethyl-1.4-pregnadien-3.20-dion in 8 ml methanolischer 0.2 N Kaliumhydroxidlösung wird 40 min bei 0 °C gerührt. Man neutralisiert mit 10 %iger Essigsäure und erhält nach der Eiswasserfällung und Aufarbeitung ein Rohprodukt, das an 50 g Kieselgel mit einem Methylenchlorid-Aceton-Gradienten (0-15 % Aceton) gereinigt wird. Ausbeute 420 mg 11β.21-Dihydroxy-17α-methoxymethoxy-6α. 16β-dimethyl-1.4-pregnadien-3.20-dion.

## Beispiel 11

A) 3.0 g 21-Acetoxy-17α-hydroxy-6α. 16β-dimethyl-4.9(11)-pregnadien-3.20-dion werden analog Beispiel 9 D) mit Formaldehyddimethylacetal umgesetzt, aufgearbeitet und gereinigt. Man erhält 1.75 g 21-Acetoxy-17α-methoxymethoxy-6α.16β-dimethyl-4.9(11)-pregnadien-3.20-dion.

B) Wie im Beispiel 1 F) beschrieben, werden 1.7 g 21-Acetoxy-17α-methoxymethoxy-6α.16β-dimethyl-4.9(11)-pregnadien-3.20-dion mit N-Bromsuccinimid umgesetzt, aufgearbeitet und gereinigt. Ausbeute 810 mg 21-Acetoxy-9α-brom-11β-hydroxy-17α-methoxymethoxy-6α.16β-dimethyl-4-pregnen-3.20-dion.

C) Unter den Bedingungen des Beispiels 1 G) werden 800 mg 21-Acetoxy-9α-brom-11β-hydroxy-17α-methoxymethoxy-6α.16β-dimethyl-4-pregnen-3.20-dion mit Tributylzinnhydrid debromiert, aufgearbeitet und gereinigt. Man isoliert 415 mg 21-Acetoxy-11β-hydroxy-17α-methoxymethoxy-6α.16β-dimethyl-4-pregnen-3.20-dion.

## Beispiel 12

Analog Beispiel 10 werden 400 mg 21-Acetoxy-11β-hydroxy-17α-methoxymethoxy-6α.16β-dimethyl-4-pregnen-3.20-dion mit methanolischer Kalilauge verseift, aufgearbeitet und gereinigt. Ausbeute 250 mg 11β.21-Dihydroxy-17α-methoxymethoxy-6α.16β-dimethyl-4-pregnen-3.20-dion.

## Beispiel 13

A) Eine Lösung von 2.4 g 9α-Brom-11β-hydroxy-6α.16β-dimethyl-17α.21-dipropionyloxy-1.4-pregnadien-3.20-dion in 34 ml Aceton wird nach Zugabe von 3.6 g Kaliumcarbonat 24 h bei Raumtemperatur gerührt. Man filtriert das Kaliumcarbonat ab und engt das Filtrat im Vakuum ein. Der Rückstand wird an 100 g Kieselgel mit einem Hexan-Essigester-Gradienten (0-40 % Essigester) gereinigt. Man isoliert 1.4 g 9.11β-Epoxy-6α.16β-dimethyl-17α.21-dipropionyloxy-1.4-pregnadien-3.20-dion. Schmp. 240-242 °C.

B) Eine auf — 40 °C abgekühlte Lösung von 3.4 ml $(HF)_n$/Pyridin wird nach Zugabe von 900 mg 9.11β-Epoxy-6α.16β-dimethyl-17α.21-dipropionyloxy-1.4-pregnadien-3.20-dion 5.75 h bei einer Temperatur zwischen — 20 °C und — 10 °C gerührt. Man gibt auf eine ammoniakalische Eiswasserlösung, filtriert den Niederschlag ab und arbeitet wie üblich auf. Das Rohprodukt wird an 100 g Kieselgel mit einem Methylenchlorid-Aceton-Gradieneten (0-10 % Aceton) gereinigt. Ausbeute 540 mg 9α-Fluor-11β-hydroxy-6α.16β-dimethyl-17.21-dipropionyloxy-1.4-pregnadien-3.20-dion.

Beispiel 14

A) 3.0 g 9α-Brom-11β-hydroxy-6α.16β-dimethyl-17α.21-dipropionyloxy-4-pregnen-3.20-dion werden analog Beispiel 13 A) mit Kaliumcarbonat behandelt und aufgearbeitet. Man isoliert 1.95 g 9.11β-Epoxy-6α.16β-dimethyl-17α.21-dipropionyloxy-4-pregnen-3.20-dion. Schmp. 220-221 °C.

B) Unter den Bedingungen des Beispiels 13 B) werden 1.6 g 9.11β-Epoxy-6α.16β-dimethyl-17α.21-dipropionyloxy-4-pregnen-3.20-dion mit $(HF)_n$/Pyridin umgesetzt, aufgearbeitet und gereinigt. Ausbeute 690 mg 9α-Fluor-11β-hydroxy-6α.16β-dimethyl-17α.21-dipropionyloxy-4-pregnen-3.20-dion. Schmp. 170-171 °C.

Beispiel 15

A) Wie im Beispiel 1 A) beschrieben, wird 1.0 g 9α-Fluor-11β.17α.21-trihydroxy-6α.16β-dimethyl-4-pregnen-3.20-dion mit Orthopropionsäuretriethylester umgesetzt und aufgearbeitet. Man isoliert 17α.21-(1-Ethoxy-propylidendioxy)-9α-fluor-11β-hydroxy-6α.16β-dimethyl-4-pregnen-3.20-dion als Öl.

B) Das rohe 17α.21-(1-Ethoxy-propylidendioxy)-9α-fluor-11β-hydroxy-6α.16β-dimethyl-4-pregnen-3.20-dion wird unter den Bedingungen des Beispiels 1 B) umgesetzt, aufgearbeitet und gereinigt. Ausbeute 795 mg 9α-Fluor-11β.21-dihydroxy-6α.16β-dimethyl-17α-propionyloxy-4-pregnen-3.20-dion.

Beispiel 16

1.0 g 9α-Fluor-11β.17α.21-trihydroxy-6α.16β-dimethyl-1.4-pregnadien-3.20-dion werden unter den Bedingungen der Beispiele 7 A) und 7 B) umgesetzt, aufgearbeitet und gereinigt. Man isoliert 805 mg 17α-Benzoyloxy-9α-fluor-11β.21-dihydroxy-6α.16β-dimethyl-1.4-pregnadien-3.20-dion.

Beispiel 17

A) Analog Beispiel 13 A) werden 1.5 g 21-Acetoxy-9α-brom-11β-hydroxy-6α.16β-dimethyl-17α-propionyloxy-4-pregnen-3.20-dion mit Kaliumcarbonat umgesetzt, aufgearbeitet und gereinigt. Ausbeute 1.05 g 21-Acetoxy-9.11β-epoxy-6α.16β-dimethyl-17α-propionyloxy-4-pregnen-3.20-dion. Schmp. 195-197 °C.

B) Unter den Bedingungen des Beispiels 13 B) werden 800 mg 21-Acetoxy-9.11β-epoxy-6α.16β-dimethyl-17α-propionyloxy-4-pregnen-3.20-dion mit 3 ml einer $(HF)_n$/Pyridin-Lösung umgesetzt und aufgearbeitet. Es werden 530 mg 21-Acetoxy-9α-fluor-11β-hydroxy-6α.16β-dimethyl-17α-propionyloxy-4-pregnen-3.20-dion erhalten. Schmp. 167-168 °C.

Beispiel 18

A) 3.0 g 9α-Brom-21-chlor-11β-hydroxy-6α.16β-dimethyl-17α-propionyloxy-4-pregnen-3.20-dion werden, wie im Beispiel 13 A) beschrieben, mit Kaliumcarbonat umgesetzt, aufgearbeitet und gereinigt. Man erhält 2.1 g 21-Chlor-9.11β-epoxy-6α.16β-dimethyl-17α-propionyloxy-4-pregnen-3.20-dion vom Schmp. 201-202 °C.

B) Unter den Bedingungen des Beispiels 13 B) werden 1.75 g 21-Chlor-9.11β-epoxy-6α.16β-dimethyl-17α-propionyloxy-4-pregnen-3.20-dion mit 6 ml einer $(HF)_n$/Pyridin-Lösung umgesetzt, aufgearbeitet und gereinigt. Ausbeute 930 mg 21-Chlor-9α-fluor-11β-hydroxy-6α.16β-dimethyl-17α-propionyloxy-4-pregnen-3.20-dion. Schmp. 234-235 °C.

Beispiel 19

A) Aus 1.7 g 9α-Brom-21-chlor-11β-hydroxy-6α.16β-dimethyl-17α-propionyloxy-1.4-pregnadien-3.20-dion werden analog Beispiel 13 A) mit Kaliumcarbonat 970 mg 21-Chlor-9.11β-epoxy-6α.16β-dimethyl-17α-propionyloxy-1.4-pregnadien-3.20-dion hergestellt. Schmp. 220-222 °C.

B) Wie im Beispiel 13 B) beschrieben, werden 830 mg 21-Chlor-9.11β-epoxy-6α.16β-dimethyl-17α-propionyloxy-1.4-pregnadien-3.20-dion mit 3.2 ml einer $(HF)_n$/Pyridin-Lösung umgesetzt, aufgearbeitet und gereinigt. Man erhält 650 mg 21-Chlor-9α-fluor-11β-hydroxy-6α.16β-dimethyl-17α-propionyloxy-1.4-pregnadien-3.20-dion. Schmp. 233-235 °C.

Beispiel 20

Zu einer Suspension von 11.1 g Kupfer (I)-jodid in 220 ml wasserfreiem Tetrahydrofuran tropft man bei 0 °C unter Argon 55 ml einer 1.6 M Lithiummethyl-Lösung. Man rührt 15 min bei 0 °C weiter und kühlt die gelbliche Lösung auf —30 °C ab. Nach tropfenweiser Zugabe einer Lösung von 8.8 g 9α-Fluor-

11β,17α-dihydroxy-6α,16β-dimethyl-21-valeryloxy-4-pregnen-3.20-dion wird das Reaktionsgemisch 40 min bei —30 °C nachgerührt, anschließend auf eine eiskalte gesättigte Ammoniumchlorid-Lösung gegeben und mit Essigester extrahiert. Die organischen Extrakte werden wie üblich aufgearbeitet und das Rohprodukt an 100 g Kieselgel mit einem Methylenchlorid-Aceton-Gradienten (0-20 % Aceton) gereinigt. Ausbeute 6.6 g 9α-Fluor-11β,21-dihydroxy-6α.16β-dimethyl-17α-valeryloxy-4-pregnen-3.20-dion.

Beispiel 21

800 mg 6α.16β-dimethyl-17α.21-dipropionyloxy-1.4.9(11)-pregnatrien-3.20-dion werden in 8.0 ml Dioxan mit 660 mg N-Chlorsuccinimid versetzt und nach tropfenweiser Zugabe von 4.0 ml einer 10 %igen Perchlorsäurelösung 3 h bei Raumtemperatur gerührt. Man gibt auf eine Eiswasser-Kochsalz-Lösung und arbeitet wie üblich auf. Ausbeute 690 mg 9α-Chlor-11β-hydroxy-6α.16β-dimethyl-17α.21-dipropionyloxy-1.4-pregnadien-3.20-dion. Schmp. 197-198 °C.

Beispiel 22

Analog Beispiel 21 werden 800 mg 21-Chlor-6α.16β-dimethyl-17α-propionyloxy-1.4.9(11)-pregnatrien-3.20-dion mit N-Chlorsuccinimid umgesetzt, aufgearbeitet und gereinigt. Man isoliert 620 mg 9α.21-Dichlor-11β-hydroxy-6α.16β-dimethyl-17α-propionyloxy-1.4-pregnadien-3.20-dion vom Schmp. 240-241 °C.

Beispiel 23

Unter den Bedingungen des Beispiels 21 werden 1.3 g 21-Chlor-6α.16β-dimethyl-17α-propionyloxy-4.9(11)-pregnadien-3.20-dion mit N-Chlorsuccinimid umgesetzt, aufgearbeitet und gereinigt. Es werden 590 mg 9α.21-Dichlor-11β-hydroxy-6,16β-dimethyl-17α-propionyloxy-4-pregnen-3.20-dion erhalten. Schmp. 197-198 °C.

Beispiel 24

Wie im Beispiel 21 beschrieben, wird 1.0 g 21-Acetoxy-6α.16β-dimethyl-17α-propionyloxy-4.9(11)-pregnadien-3.20-dion mit N-Chlorsuccinimid umgesetzt, aufgearbeitet und gereinigt. Ausbeute 540 mg 21-Acetoxy-9α-chlor-11β-hydroxy-17α-propionyloxy-6α.16β-dimethyl-4-pregnen-3.20-dion. Schmp. 190-192 °C.

Beispiel 25

A) Unter den Bedingungen des Beispiels 1 A) wird 1.0 g 9α-Chlor-11β.17α.21-trihydroxy-6α.16β-dimethyl-1.4-pregnadien-3.20-dion mit Orthoessigsäuretriethylester umgesetzt und aufgearbeitet. Es wird 9α-Chlor-17α.21-(1-ethoxy-ethylidendioxy)-11β-hydroxy-6α.16β-dimethyl-1.4-pregnadien-3.20-dion als Öl isoliert.

B) Das rohe 9α-Chlor-17α.21-(1-ethoxy-ethylidendioxy)-11β-hydroxy-6α.16β-dimethyl-1.4-pregnadien-3.20-dion wird analog Beispiel 1 B) hydrolysiert, aufgearbeitet und gereinigt. Man isoliert 860 mg 17α-Acetoxy-9α-chlor-11β.21-dihydroxy-6α.16β-dimethyl-1.4-pregnadien-3.20-dion.

Beispiel 26

A) Analog Beispiel 1 A) wird 1.0 g 9α-Chlor-11β.17α.21-trihydroxy-6α.16β-dimethyl-1.4-pregnadien-3.20-dion mit Orthopropionsäuretriethylester umgesetzt und aufgearbeitet. Man isoliert 9α-Chlor-17α.21-(1-ethoxy-propylidendioxy)-11β-hydroxy-6α.16β-dimethyl-1.4-pregnadien-3.20-dion als Öl.

B) 9α-Chlor-17α.21-(1-ethoxy-propylidendioxy)-11β-hydroxy-6α.16β-dimethyl-1.4-pregnadien-3.20-dion werden als Rohprodukt unter den Bedingungen des Beispiels 1 B) hydrolysiert, aufgearbeitet und gereinigt. Ausbeute 810 mg 9α-Chlor-11β.21-dihydroxy-6α.16β-dimethyl-17α-propionyloxy-1.4-pregnadien-3.20-dion.

Beispiel 27

A) Unter den Bedingungen des Beispiels 1 A) wird 1.0 g 9α-Chlor-11β.17α.21-trihydroxy-6α.16β-dimethyl-1.4-pregnadien-3.20-dion mit Orthobenzoesäuretriethylester umgesetzt und aufgearbeitet. Es wird das 9α-Chlor-17α.21-(1-ethoxy-benzylidendioxy)-11β-hydroxy-6α.16β-dimethyl-1.4-pregnadien-3.20-dion als Öl isoliert.

B) Das rohe 9α-Chlor-17α.21-(1-ethoxy-benzylidendioxy)-11β-hydroxy-6α.16β-dimethyl-1.4-pregnadien-3.20-dion wird, wie im Beispiel 1 B) beschrieben, hydrolysiert, aufgearbeitet und gereinigt. Man isoliert 710 mg 17α-Benzoyloxy-9α-chlor-11β.21-dihydroxy-6α.16β-dimethyl-1.4-pregnadien-3.20-dion.

Beispiel 28

A) 1.0 g 9α-Chlor-11β.17α.21-trihydroxy-6α.16β-dimethyl-1.4-pregnadien-3.20-dion werden analog Beispiel 1 A) mit Orthobuttersäuretriethylester umgesetzt und aufgearbeitet. Man isoliert 9α-Chlor-17α.21-(1-ethoxy-butylidendioxy)-11β-hydroxy-6α.16β-dimethyl-1.4-pregnadien-3.20-dion als Öl.

B) Das rohe 9α-Chlor-17α.21-(1-ethoxy-butylidendioxy)-11β-hydroxy-6α.16β-dimethyl-1.4-pregnadien-3.20-dion wird unter den Bedingungen des Beispiels 1 B) hydrolysiert, aufgearbeitet und gereinigt. Ausbeute 830 mg 17α-Butyryloxy-9α-chlor-11β.21-dihydroxy-6α.16β-dimethyl-1.4-pregnadien-3.20-dion.

Beispiel 29

1.1 g 17α-Butyryloxy-9α-chlor-11β.21-dihydroxy-6α.16β-dimethyl-1.4-pregnadien-3.20-dion werden in 10 ml Pyridin und 5 ml Acetanhydrid 1.5 h bei Raumtemperatur gerührt und anschließend auf Eiswasser gegeben. Nach der üblichen Aufarbeitung wird das Rohprodukt an 100 g Kieselgel mit einem Methylenchlorid-Aceton-Gradienten (0-25 % Aceton) gereinigt. Man isoliert 960 mg 21-Acetoxy-17α-butyryloxy-9α-chlor-11β-hydroxy-6α.16β-dimethyl-1.4-pregnadien-3.20-dion.

Beispiel 30

Unter den Bedingungen des Beispiels 29 werden 1.5 g 9α-Chlor-11β.21-dihydroxy-6α.16β-dimethyl-17α-propionyloxy-1.4-pregnadien-3.20-dion mit Acetanhydrid umgesetzt, aufgearbeitet und gereinigt. Ausbeute 1.4 g 21-Acetoxy-9α-chlor-11β-hydroxy-6α.16β-dimethyl-17α-propionyloxy-1.4-pregnadien-3.20-dion.

Beispiel 31

A) 4.0 g 21-Acetoxy-17α-hydroxy-6α·16β-dimethyl-1.4.9(11)-pregnatrien-3.20-dion werden analog Beispiel 9D) mit 36 ml Formaldehyddiäthylacetal umgesetzt, aufgearbeitet und gereinigt. Man isoliert 2.0 g 21-Acetoxy-17α-ethoxymethoxy-6α·16β-dimethyl-1.4.9-pregnatrien-3.20-dion.

B) Unter den Bedingungen des Beispiels 4B) werden 2.0 g 21-Acetoxy-17α-ethoxymethoxy-6α·16β-dimethyl-1.4.9-pregnatrien-3.20-dion mit N-Bromsuccinimid umgesetzt und aufgearbeitet. Ausbeute 2.2 g 21-Acetoxy-9α-brom-17α-ethoxymethoxy-11β-hydroxy-6α·16β-dimethyl-1.4-pregnadien-3.20-dion.

C) Analog Beispiel 1G) werden 2.2 g 21-Acetoxy-9α-brom-17α-ethoxymethoxy-11β-hydroxy-6α·16β-dimethyl-1.4-pregnadien-3.20-dion mit Tributylzinnhydrid enthalogeniert, aufgearbeitet und gereinigt. Man erhält 1.3 g 21-Acetoxy-17α-ethoxymethoxy-11β-hydroxy-6α·16β-dimethyl-1.4-pregnadien-3.20-dion. Schmelzpunkt 137-139 °C.

Beispiel 32

Eine Suspension von 0.7 g 21-Acetoxy-17α-ethoxymethoxy-11β-hydroxy-6α·16β-dimethyl-1.4-pregnadien-3.20-dion in 8 ml einer 0.2 N methanolischen Kaliumhydroxidlösung wird 30 Minuten bei 0 °C gerührt und anschließend mit 10 proz. Essigsäure neutralisiert. Nach der üblichen Aufarbeitung isoliert man 470 mg 17α-Ethoxymethoxy-11β·21-dihydroxy-6α·16β-dimethyl-1.4-pregnadien-3.20-dion. Schmelzpunkt 100-102 °C.

Beispiel 33

A) Eine Lösung von 1.0 g 21-Acetoxy-17α-hydroxy-6α·16β-dimethyl-1.4.9-pregnatrien-3.20-dion in 13 ml Diethylenglykoldimethylether und 1.5 ml Essigsäureanhydrid wird mit 1.5 g N.N-Dimethylaminopyridin 31 Stunden bei 80 °C gerührt. Nach der Eiswasserfällung wird wie üblich aufgearbeitet und an 100 g Kieselgel mit einem Methylenchlorid/Aceton-Gradienten chromatographiert. Man isoliert 730 mg 17α·21-Diacetoxy-6α·16β-dimethyl-1.4.9-pregnatrien-3.20-dion. Schmelzpunkt 134-136 °C.

B) Analog Beispiel 4B) werden 500 mg 17α·21-Diacetoxy-6α·16β-dimethyl-1.4.9-pregnatrien-3.20-dion mit N-Bromsuccinimid umgesetzt, aufgearbeitet und gereinigt. Ausbeute 565 mg 17α·21-Diacetoxy-9α-brom-11β-hydroxy-6α·16β-dimethyl-1.4-pregnadien-3.20-dion.
Schmelzpunkt 205-206 °C.

C) Wie im Beispiel 1G) beschrieben, werden 460 mg 17α·21-Diacetoxy-9α-brom-11β-hydroxy-6α·16β-dimethyl-1.4-pregnadien-3.20-dion mit Tributylzinnhydrid debromiert und aufbereitet. Man erhält 400 mg 17α·21-Diacetoxy-11β-hydroxy-6α·16β-dimethyl-1.4-pregnadien-3.20-dion.
Schmelzpunkt 124-126 °C.

Beispiel 34

A) Unter den Bedingungen des Beispiels 33A) wird 1.0 g 21-Acetoxy-17α-hydroxy-6α·16β-dimethyl-1.4.9-pregnatrien-3.20-dion mit Propionsäureanhydrid behandelt und aufgearbeitet. Nach der Reinigung isoliert man 750 mg 21-Acetoxy-6α·16β-dimethyl-17α-propionyloxy-1.4.9-pregnatrien-3.20-dion.
Schmelzpunkt 110-112 °C.

B) 800 mg 21-Acetoxy-6α·16β-dimethyl-17α-propionyloxy-1.4.9-pregnatrien-3.20-dion werden analog Beispiel 4B) mit N-Bromsuccinimid umgesetzt und aufgearbeitet. Ausbeute 850 mg 21-Acetoxy-9α-brom-11β-hydroxy-6α·16β-dimethyl-17α-propionyloxy-1.4-pregnadien-3.20-dion. Schmelzpunkt 185-187 °C.

C) Die Debromierung der 800 mg 21-Acetoxy-9α-brom-11β-hydroxy-6α·16β-dimethyl-17α-propionyloxy-1.4-pregnadien-3.20-dion wird analog Beispiel 1G) mit Tributylzinnhydrid durchgeführt. Nach der Aufreinigung isoliert man 520 mg 21-Acetoxy-11β-hydroxy-6α·16β-dimethyl-17α-propionyloxy-1.4-pregnadien-3.20-dion. Schmelzpunkt 115-117 °C.

Beispiel 35

A) Wie im Beispiel 33A) beschrieben, wird 1.0 g 21-Acetoxy-17α-hydroxy-6α·16β-dimethyl-1.4.9-pregnatrien-3.20-dion mit Buttersäureanhydrid umgesetzt, aufgearbeitet und gereinigt. Man erhält 860 mg 21-Acetoxy-17α-butyryloxy-6α·16β-dimethyl-1.4.9-pregnatrien-3.20-dion. Schmelzpunkt 88-90 °C.

B) 800 mg 21-Acetoxy-17α-butyryloxy-6α·16β-dimethyl-1.4.9-pregnatrien-3.20-dion werden unter den Bedingungen des Beispiels 4B) mit N-Bromsuccinimid umgesetzt, aufgearbeitet und gereinigt. Ausbeute 830 mg 21-Acetoxy-9α-brom-17α-butyryloxy-11β-hydroxy-6α·16β-dimethyl-1.4-pregnadien-3.20-dion. Schmelzpunkt 173-175 °C.

C) Analog Beispiel 1G) werden 780 mg 21-Acetoxy-9α-brom-17α-butyryloxy-11β-hydroxy-6α·16β-dimethyl-1.4-pregnadien-3.20-dion mit Tributylzinnhydrid debromiert, aufgearbeitet und gereinigt. Man isoliert 610 mg 21-Acetoxy-17α-butyryloxy-11β-hydroxy-6α·16β-dimethyl-1.4-pregnadien-3.20-dion. Schmelzpunkt 106-108 °C.

**Patentansprüche**

1. 6,16-Dimethylkortikoide der allgemeinen Formel I

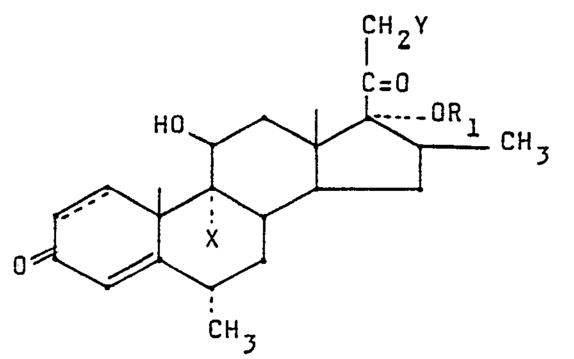

(I)

worin die Bindung ⋯ eine Einfachbindung oder eine Doppelbindung darstellt und
X ein Wasserstoffatom, ein Fluoratom, oder ein Chloratom
$R_1$ eine Formylgruppe, eine 2 bis 8 Kohlenstoffatome enthaltende Alkanoylgruppe oder Alkoxyalkylgruppe oder eine Benzoylgruppe und
Y ein Chloratom, eine Hydroxygruppe, eine Formylgruppe, eine 2 bis 8 Kohlenstoffatome enthaltende Alkanoyloxygruppe oder eine Benzoyloxygruppe bedeuten.

2. 21-Acetoxy-11β-hydroxy-6α,16β-dimethyl-17α-propionyloxy-4-pregnen-3,20-dion,
11β-Hydroxy-6α,16β-dimethyl-17α,21-dipropionyloxy-4-pregnen-3,20-dion,
21-Chlor-11β-hydroxy-6α,16β-dimethyl-17α-propionyloxy-4-pregnen-3,20-dion,
11β-Hydroxy-6α,16β-dimethyl-17α,21-dipropionyloxy-1,4-pregnadien-3,20-dion,
21-Chlor-11β-hydroxy-6α,16β-dimethyl-17α-propionyloxy-1,4-pregnadien-3,20-dion und
21-Acetoxy-11β-hydroxy-6α,16β-dimethyl-17α-propionyloxy-1,4-pregnadien-3,20-dion.

3. 17α-Butyryloxy-11β,21-dihydroxy-6α,16β-dimethyl-4-pregnen-3,20-dion und
21-Acetoxy-17α-butyryloxy-11β-hydroxy-6α,16β-dimethyl-1,4-pregnadien-3,20-dion.
4. 11β-Hydroxy-6α,16β-dimethyl-17α,21-dipropionyloxy-4-pregnen-3,20-dion.
5. 17α-Acetoxy-11β,21-dihydroxy-6α,16β-dimethyl-4-pregnen-3,20-dion und
17α,21-Diacetoxy-11β-hydroxy-6α,16β-dimethyl-1,4-pregnadien-3,20-dion.
6. 21-Acetoxy-11β-hydroxy-17α-methoxymethoxy-6α,16β-dimethyl-1,4-pregnadien-3,20-dion,
21-Acetoxy-11β-hydroxy-17α-methoxymethoxy-6α,16β-dimethyl-4-pregnen-3,20-dion,
11β,21-Dihydroxy-17α-methoxymethoxy-6α,16β-dimethyl-4-pregnen-3,20-dion,
11β,21-Dihydroxy-17α-methoxymethoxy-6α,16β-dimethyl-1,4-pregnadien-3,20-dion,
21-Acetoxy-17α-ethoxymethoxy-11β-hydroxy-6α,16β-dimethyl-1,4-pregnadien-3,20 dion und
17α-Ethoxymethoxy-11β,21-dihydroxy-6α,16β-dimethyl-1,4-pregnadien-3,20-dion.
7. 9α-Fluor-11β-hydroxy-6α,16β-dimethyl-17α,21-dipropionyloxy-1,4-pregnadien-3,20-dion,
9α-Fluor-11β-hydroxy-6α,16β-dimethyl-17α,21-dipropionyloxy-4-pregnen-3,20-dion,
9α-Fluor-11β,21-dihydroxy-6α,16β-dimethyl-17α-propionyloxy-4-pregnen-3,20-dion,
17α-Benzoyloxy-9α-fluor-11β,21-dihydroxy-6α,16β-dimethyl-1,4-pregnadien-3,20-dion,
21-Acetoxy-9α-fluor-11β-hydroxy-6α,16β-dimethyl-17α-propionyloxy-4-pregnen-3,20-dion,
21-Chlor-9α-fluor-11β-hydroxy-6α,16β-dimethyl-17α-propionyloxy-4-pregnen-3,20-dion,
21-Chlor-9α-fluor-11β-hydroxy-6α,16β-dimethyl-17α-propionyloxy-1,4-pregnadien-3,20-dion und
9α-Fluor-11β,21-dihydroxy-6α,16β-dimethyl-17α-valeryloxy-4-pregnen-3,20-dion.
8. 9α-Chlor-11β-hydroxy-6α,16β-dimethyl-17α,21-dipropionyloxy-1,4-pregnadien-3,20-dion,
9α,21-Dichlor-11β-hydroxy-6α,16β-dimethyl-17α-propionyloxy-1,4-pregnadien-3,20-dion,
9α,21-Dichlor-11β-hydroxy-6α,16β-dimethyl-17α-propionyloxy-4-pregnen-3,20-dion,
21-Acetoxy-9α-chlor-11β-hydroxy-6α,16β-dimethyl-17α-propionyloxy-4-pregnen-3,20-dion,
17α-Acetoxy-9α-chlor-11β,21-dihydroxy-6α,16β-dimethyl-1,4-pregnadien-3,20-dion,
9α-Chlor-11β,21-dihydroxy-6α,16β-dimethyl-17α-propionyloxy-1,4-pregnadien-3,20-dion,
17α-Benzoyloxy-9α-chlor-11β,21-dihydroxy-6α,16β-dimethyl-1,4-pregnadien-3,20-dion,
17α-Butyryloxy-9α-chlor 11β,21-dihydroxy-6α,16β-dimethyl-1,4-pregnadien-3,20-dion,
21-Acetoxy-17α-butyryloxy-9α-chlor-11β-hydroxy-6α,16β-dimethyl-1,4-pregnadien-3,20-dion und
21-Acetoxy-9α-chlor-11β-hydroxy-6α,16β-dimethyl-17α-propionyloxy-1,4-pregnadien-3,20-dion.

9. Pharmazeutische Präparate gekennzeichnet durch den Gehalt von ein oder zwei 6,16-Dimethylkortikoiden gemäß Patentanspruch 1 bis 8.

10. Verfahren zur Herstellung von 6,16-Dimethylkortikoiden der allgemeinen Formel I gemäß Patentanspruch 1, dadurch gekennzeichnet, daß man in an sich bekannter Weise

a) den Epoxidring eines Steroides der allgemeinen Formel II

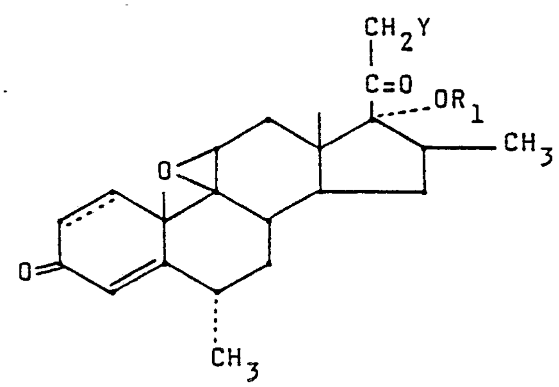

(II)

worin ----, $R_1$ und Y die im Patentanspruch 1 genannte Bedeutung besitzen, mit Fluorwasserstoff öffnet oder

b) an die $\Delta^{9(11)}$-Doppelbindung eines Steroids der allgemeinen Formel III

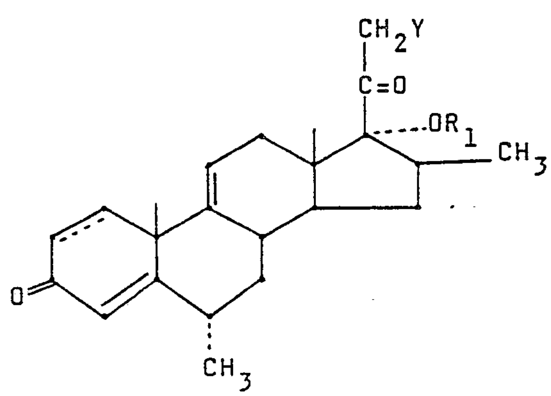

(III)

worin ....., $R_1$ und Y die im Patentanspruch 1 genannte Bedeutung besitzen unterchlorige oder unterbromige Säure anlagert und gewünschtenfalls aus den 9-Chlor- oder 9-Bromsteroiden der allgemeinen Formel I das in der 9-Position ständige Halogenatom eliminiert, 21-Acetoxysteroide der allgemeinen Formel I verseift oder 21-Hydroxysteroide der allgemeinen Formel I verestert.

11. Steroide der allgemeinen Formel VI

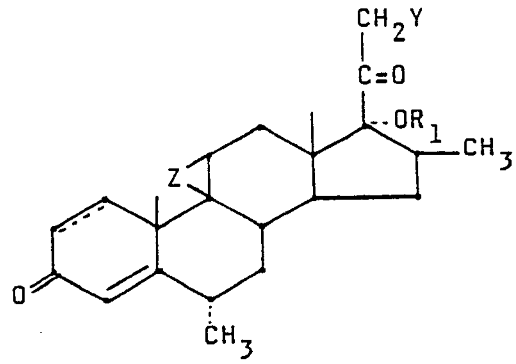

(VI)

worin ....., $R_1$ und Y die im Patentanspruch 1 genannte Bedeutung besitzen

Z eine Kohlenstoff-Kohlenstoff-Bindung oder ein Sauerstoffatom bedeutet und worin
$R_4$ und $R_5$ gemeinsam eine Methylengruppe oder
$R_4$ ein Wasserstoffatom oder eine Methylgruppe und
$R_5$ ein Wasserstoffatom darstellen.

12. 21-Acetoxy-6α,16β-dimethyl-17α-propionyloxy-1,4,9-pregnatrien-3,20-dion
21-Acetoxy-6α,16β-dimethyl-17α-propionyloxy-4,9(11)-pregnadien-3,20-dion
6α,16β-Dimethyl-17α,21-dipropionyloxy-4,9(11)-pregnadien-3,20-dion,
21-Chlor-6α,16β-dimethyl-17α-propionyloxy-4,9(11)-pregnadien-3,20-dion,
6α,16β-Dimethyl-17α,21-dipropionyloxy-1,4,9(11)-pregnatrien-3,20-dion,
21-Chlor-6α,16β-dimethyl-17α-propionyloxy-1,4,9(11)-pregnatrien-3,20-dion,

13. 9,11β-Epoxy-6α,16β-dimethyl-17α,21-dipropionyloxy-1,4-pregnadien-3,20-dion,
9,11β-Epoxy-6α,16β-dimethyl-17α,21-dipropionyloxy-4-pregnen-3,20-dion,
21-Acetoxy-9,11β-epoxy-6α,16β-dimethyl-17α-propionyloxy-4-pregnen-3,20-dion,
21-Chlor-9,11β-epoxy-6α,16β-dimethyl-17α-propionyloxy-4-pregnen-3,20-dion und
21-Chlor-9,11β-epoxy-6α,16β-dimethyl-17α-propionyloxy-1,4-pregnadien-3,20-dion.

## Claims

1. 6,16-dimethyl corticoids of the general formula I

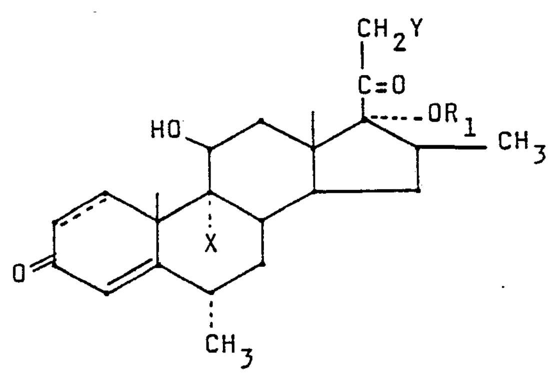

(I)

in which

the bond ..... represents a single bond or a double bond,
and

X represents a hydrogen atom, a fluorine atom or a chlorine atom,

$R_1$ represents a formyl group, an alkanoyl group or alkoxyalkyl group containing from 2 to 8 carbon atoms, or a benzoyl group, and

Y represents a chlorine atom, a hydroxy group, a formyl group, an alkanoyloxy group containing from 2 to 8 carbon atoms, or a benzoyloxy group.

2. 21-Acetoxy-11β-hydroxy-6α,16β-dimethyl-17α-propionyloxy-4-pregnene-3,20-dione,
11β-hydroxy-6α,16β-dimethyl-17α,21-dipropionyloxy-4-pregnene-3,20-dione,
21-chloro-11β-hydroxy-6α,16β-dimethyl-17α-propionyloxy-4-pregnene-3,20-dione,
11β-hydroxy-6α,16β-dimethyl-17α,21-dipropionyloxy-1,4-pregnadiene-3,20-dione,
21-chloro-11β-hydroxy-6α,16β-dimethyl-17α-propionyloxy-1,4-pregnadiene-3,20-dione and
21-acetoxy-11β-hydroxy-6α,16β-dimethyl-17α-propionyloxy-1,4-pregnadiene-3,20-dione.

3. 17α-Butyryloxy-11β,21-dihydroxy-6α,16β-dimethyl-4-pregnene-3,20-dione and
21-acetoxy-17α-butyryloxy-11β-hydroxy-6α,16β-dimethyl-1,4-pregnadiene-3,20-dione.

4. 11β-Hydroxy-6α,16β-dimethyl-17α,21-dipropionyloxy-4-pregnene-3,20-dione.

5. 17α-Acetoxy-11β,21-dihydroxy-6α,16β-dimethyl-4-pregnene-3,20-dione and
17α,21-diacetoxy-11β-hydroxy-6α,16β-dimethyl-1,4-pregnadiene-3,20-dione.

6. 21-Acetoxy-11β-hydroxy-17α-methoxymethoxy-6α,16β-dimethyl-1,4-pregnadiene-3,20-dione,
21-acetoxy-11β-hydroxy-17α-methoxymethoxy-6α,16β-dimethyl-4-pregnene-3,20-dione,
11β,21-dihydroxy-17α-methoxymethoxy-6α,16β-dimethyl-4-pregnene-3,20-dione,
11β,21-dihydroxy-17α-methoxymethoxy-6α,16β-dimethyl-1,4-pregnadiene-3,20-dione,
21-acetoxy-17α-ethoxymethoxy-11β-hydroxy-6α,16β-dimethyl-1,4-pregnadiene-3,20-dione and
17α-ethoxymethoxy-11β,21-dihydroxy-6α,16β-dimethyl-1,4-pregnadiene-3,20-dione.

7. 9α-Fluoro-11β-hydroxy-6α,16β-dimethyl-17α,21-dipropionyloxy-1,4-pregnadiene-3,20-dione,
9α-fluoro-11β-hydroxy-6α,16β-dimethyl-17α,21-dipropionyloxy-4-pregnene-3,20-dione,
9α-fluoro-11β,21-dihydroxy-6α,16β-dimethyl-17α-propionyloxy-4-pregnene-3,20-dione,
17α-benzoyloxy-9α-fluoro-11β,21-dihydroxy-6α,16β-dimethyl-1,4-pregnadiene-3,20-dione,
21-acetoxy-9α-fluoro-11β-hydroxy-6α,16β-dimethyl-17α-propionyloxy-4-pregnene-3,20-dione,
21-chloro-9α-fluoro-11β-hydroxy-6α,16β-dimethyl-17α-propionyloxy-4-pregnene-3,20-dione,
21-chloro-9α-fluoro-11β-hydroxy-6α,16β-dimethyl-17α-propionyloxy-1,4-pregnadiene-3,20-dione,
and
9α-fluoro-11β,21-dihydroxy-6α,16β-dimethyl-17α-valeryloxy-4-pregnene-3,20-dione.

8. 9α-Chloro-11β-hydroxy-6α,16β-dimethyl-17α,21-dipropionyloxy-1,4-pregnadiene-3,20-dione,
9α,21-dichloro-11β-hydroxy-6α,16β-dimethyl-17α-propionyloxy-1,4-pregnadiene-3,20-dione,
9α,21-dichloro-11β-hydroxy-6α,16β-dimethyl-17α-propionyloxy-4-pregnene-3,20-dione,
21-acetoxy-9α-chloro-11β-hydroxy-6α,16β-dimethyl-17α-propionyloxy-4-pregnene-3,20-dione,
17α-acetoxy-9α-chloro-11β,21-dihydroxy-6α,16β-dimethyl-1,4-pregnadiene-3,20-dione,
9α-chloro-11β,21-dihydroxy-6α,16β-dimethyl-17α-propionyloxy-1,4-pregnadiene-3,20-dione,
17α-benzoyloxy-9α-chloro-11β,21-dihydroxy-6α,16β-dimethyl-1,4-pregnadiene-3,20-dione,
17α-butyryloxy-9α-chloro-11β,21-dihydroxy-6α,16β-dimethyl-1,4-pregnadiene-3,20-dione,
21-acetoxy-17α-butyryloxy-9α-chloro-11β-hydroxy-6α,16β-dimethyl-1,4-pregnadiene-3,20-dione and
21-acetoxy-9α-chloro-11β-hydroxy-6α,16β-dimethyl-17α-propionyloxy-1,4-pregnadiene-3,20-dione.

9. Pharmaceutical preparations characterised by a content of one or two 6,16-dimethyl corticoids according to claims 1 to 8.

10. Process for the manufacture of 6,16-dimethyl corticoids of the general formula I according to claim 1, characterised in that, in a manner known per se,

a) the epoxide ring of a steroid of the general formula II

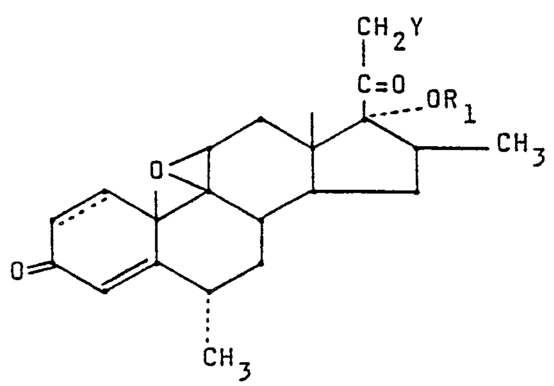

(II)

in which ....., $R_1$ and Y have the meanings given in claim 1, is opened with hydrogen fluoride, or

b) hypochlorous or hypobromous acid is added to the $\Delta^{9(11)}$-double bond of a steroid of the general formula III

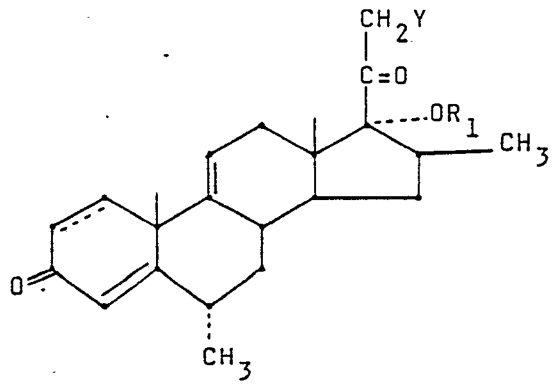
(III)

in which - - - -, $R_1$ and Y have the meanings given in claim 1, and, if desired, the halogen atom standing in the 9-position is eliminated from the 9-chloro- or 9-bromosteroids of the general formula I, 21-acetoxy steroids of the general formula I are hydrolysed or 21-hydroxy steroids of the general formula I are esterified.

11. Steroids of the general formula VI

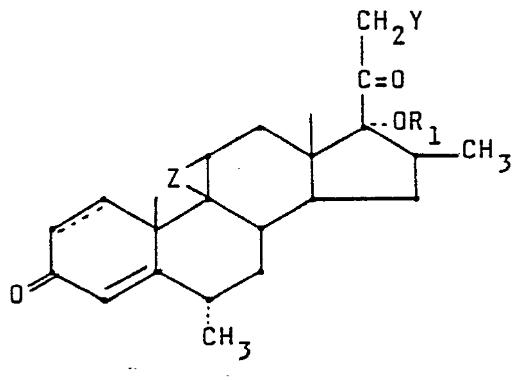
(VI)

in which - - - -, $R_1$ and Y have the meanings given in claim 1,
Z represents a carbon-carbon bond or an oxygen atom, and
$R_4$ and $R_5$ together represent a methylene group, or
$R_4$ represents a hydrogen atom or a methyl group and
$R_5$ represents a hydrogen atom.

12. 21-Acetoxy-6α,16β-dimethyl-17α-propionyloxy-1,4,9-pregnatriene-3,20-dione,
21-acetoxy-6α,16β-dimethyl-17α-propionyloxy-4,9(11)-pregnadiene-3,20-dione,
6α,16β-dimethyl-17α,21-dipropionyloxy-4,9(11)-pregnadiene-3,20-dione,
21-chloro-6α,16β-dimethyl-17α-propionyloxy-4,9(11)-pregnadiene-3,20-dione,
6α,16β-dimethyl-17α,21-dipropionyloxy-1,4,9(11)-pregnatriene-3,20-dione,
21-chloro-6α,16β-dimethyl-17α-propionyloxy-1,4,9(11)-pregnatriene-3,20-dione.

13. 9,11β-Epoxy-6α,16β-dimethyl-17α,21-dipropionyloxy-1,4-pregnadiene-3,20-dione,
9,11β-epoxy-6α,16β-dimethyl-17α,21-dipropionyloxy-4-pregnene-3,20-dione,
21-acetoxy-9,11β-epoxy-6α,16β-dimethyl-17α-propionyloxy-4-pregnene-3,20-dione,
21-chloro-9,11β-epoxy-6α,16β-dimethyl-17α-propionyloxy-4-pregnene-3,20-dione and
21-chloro-9,11β-epoxy-6α,16β-dimethyl-17α-propionyloxy-1,4-pregnadiene-3,20-dione.

**Revendications**

1. Diméthyl-6,16 corticoïdes répondant à la formule générale I :

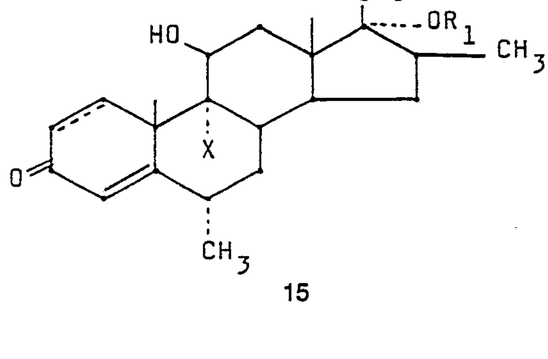
(I) .

dans laquelle :

la liaison ···· représente une liaison simple ou une liaison double,

X représente un atome d'hydrogène, de fluor ou de chlore,

$R^1$ représente un radical formyle, un radical alcoxy-alkyle ou alcanoyle contenant de 2 à 8 atomes de carbone ou un radical benzoyle, et

Y représente un atome de chlore, un radical hydroxy, un radical formyle, un radical alcanoyloxy contenant de 2 à 8 atomes de carbone ou un radical benzoyloxy.

2. Acétoxy-21 hydroxy-11β diméthyl-6α,16β propionyloxy-17α prégnène-4 dione-3,20,
hydroxy-11β diméthyl-6α,16β dipropionyloxy-17α,21 prégnène-4 dione-3,20,
chloro-21 hydroxy-11β diméthyl-6α,16β propionyloxy-17α prégnène-4 dione-3,20,
hydroxy-11β diméthyl-6α,16β dipropionyloxy-17α,21 prégnadiène-1,4 dione-3,20,
chloro-21 hydroxy-11β diméthyl-6α,16β propionyloxy-17α prégnadiène-1,4 dione-3,20
et
acétoxy-21 hydroxy-11β diméthyl-6α,16β propionyloxy-17α prégnadiène-1,4 dione-3,20.

3. Butyryloxy-17α dihydroxy-11β,21 diméthyl-6α,16β prégnène-4 dione-3,20 et
acétoxy-21 butyryloxy-17α hydroxy-11β diméthyl-6α,16β prégnadiène-1,4 dione-3,20.

4. Hydroxy-11β diméthyl-6α,16β dipropionyloxy-17α,21 prégnène-4 dione-3,20.

5. Acétoxy-17α dihydroxy-11β,21 diméthyl-6α,16β prégnène-4 dione-3,20 et diacétoxy-17α,21 hydroxy-11β diméthyl-6α,16β prégnadiène-1,4 dione-3,20.

6. Acétoxy-21 hydroxy-11β méthoxyméthoxy-17α diméthyl-6α,16β prégnadiène-1,4 dione-3,20,
acétoxy-21 hydroxy-11β méthoxyméthoxy-17α diméthyl-6α,16β prégnène-4 dione-3,20,
dihydroxy-11β,21 méthoxyméthoxy-17α diméthyl-6α,16β prégnène-4 dione-3,20,
dihydroxy-11β,21 méthoxyméthoxy-17α diméthyl-6α,16β prégnadiène-1,4 dione-3,20,
acétoxy-21 éthoxyméthoxy-17α hydroxy-11β diméthyl-6α,16β prégnadiène-1,4 dione-3,20 et éthoxyméthoxy-17α dihydroxy-11β,21 diméthyl-6α,16β prégnadiène-1,4 dione-3,20.

7. Fluoro-9α hydroxy-11β diméthyl-6α,16β dipropionyloxy-17α,21 prégnadiène-1,4 dione-3,20, fluoro-9α hydroxy-11β diméthyl-6α,16β dipropionyloxy-17α,21 prégnène-4 dione-3,20,
fluoro-9α dihydroxy-11β,21 diméthyl-6α,16β propionyloxy-17α prégnène-4 dione-3,20, benzoyloxy-17α fluoro-9α dihydroxy-11β,21 diméthyl-6α,16β prégnadiène-1,4 dione-3,20,
acétoxy-21 fluoro-9α hydroxy-11β diméthyl-6α,16β propionyloxy-17α prégnène-4 dione-3,20,
chloro-21 fluoro-9α hydroxy-11β diméthyl-6α,16β propionyloxy-17α prégnène-4 dione-3,20,
chloro-21 fluoro-9α hydroxy-11β diméthyl-6α,16β propionyloxy-17α prégnadiène-1,4 dione-3,20 et
fluoro-9α dihydroxy-11β,21 diméthyl-6α,16β valéryloxy-17α prégnène-4 dione-3,20.

8. Chloro-9α hydroxy-11β diméthyl-6α,16β dipropionyloxy-17α,21 prégnadiène-1,4 dione-3,20, dichloro-9α,21 hydroxy-11β diméthyl-6α,16β propionyloxy-17α prégnadiène-1,4 dione-3,20,
dichloro-9α,21 hydroxy-11β diméthyl-6α,16β propionyloxy-17α prégnène-4 dione-3,20,
acétoxy-21 chloro-9α hydroxy-11β diméthyl-6α,16β propionyloxy-17α prégnène-4 dione-3,20,
acétoxy-17α chloro-9α dihydroxy-11β,21 diméthyl-6α,16β prégnadiène-1,4 dione-3,20,
chloro-9α dihydroxy-11β,21 diméthyl-6α,16β propionyloxy-17α prégnadiène-1,4 dione-3,20,
benzoyloxy-17α chloro-9α dihydroxy-11β,21 diméthyl-6α,16β prégnadiène-1,4 dione-3,20,
butyryloxy-17α chloro-9α dihydroxy-11β,21 diméthyl-6α,16β prégnadiène-1,4 dione-3,20,
acétoxy-21 butyryloxy-17α chloro-9α hydroxy-11β diméthyl-6α,16β prégnadiène-1,4 dione-3,20 et
acétoxy-21 chloro-9α hydroxy-11β diméthyl-6α,16β propionyloxy-17α prégnadiène-1,4 dione-3,20.

9. Compositions pharmaceutiques caractérisées en ce qu'elles contiennent un ou deux diméthyl-6,16 corticoïdes selon l'une quelconque des revendications 1 à 8.

10. Procédé de préparation de diméthyl-6,16 corticoïdes de formule générale I selon la revendication 1, procédé caractérisé en ce que, en opérant de manière connue :

a) on ouvre, au moyen du fluorure d'hydrogène, le cycle époxy d'un stéroïde répondant à la formule générale II :

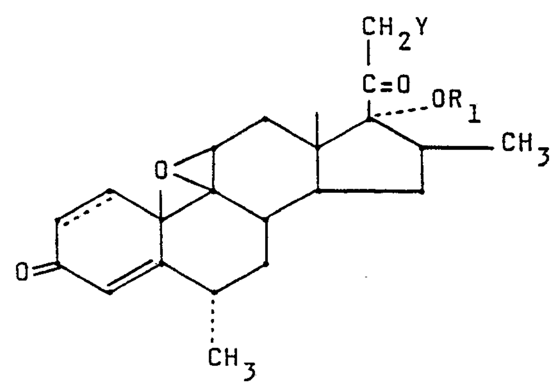

(II)

dans laquelle ...., $R^1$ et Y ont les significations données à la revendication 1, ou

b) sur la double liaison $\Delta^{9(11)}$ d'un stéroïde répondant à la formule générale III :

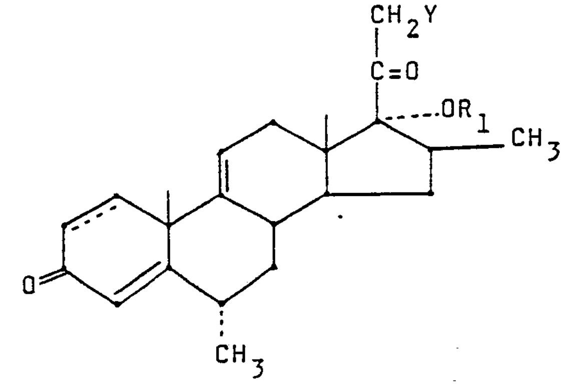

(III)

dans laquelle ...., $R^1$ et Y ont les significations données à la revendication 1, on fixe l'acide hypochloreux ou l'acide hypobromeux et, si on le désire, on élimine, des chloro-9 ou bromo-9 stéroïdes de formule générale I, l'atome d'halogène qui se trouve à la position 9, on saponifie des acyloxy-21 stéroïdes de formule générale I ou on estérifie des hydroxy-21 stéroïdes de formule générale I.

11. Stéroïdes répondant à la formule générale VI :

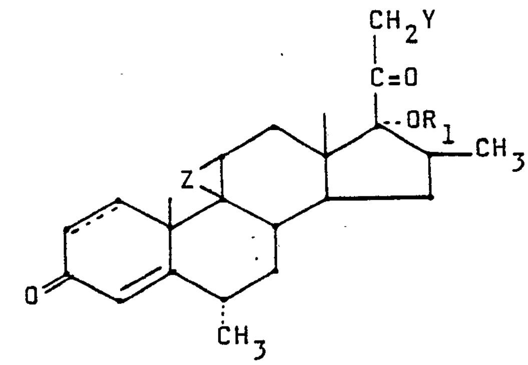

(VI)

dans laquelle

.... $R^1$ et Y ont les significations qui leur ont été données à la revendication 1, et

Z représente une liaison carbone-carbone ou un atome d'oxygène, et dans laquelle $R_4$ et $R_5$ forment ensemble un radical méthylène, ou $R_4$ représente un atome d'hydrogène ou un méthyle et $R_5$ un atome d'hydrogène.

12. Acétoxy-21 diméthyl-6α,16β propionyloxy-17α prégnatriène-1,4,9 dione-3,20,
acétoxy-21 diméthyl-6α,16β propionyloxy-17α prégnadiène-4,9(11) dione-3,20,
diméthyl-6α,16β dipropionyloxy-17α,21 prégnadiène-4,9(11) dione-3,20,
chloro-21 diméthyl-6α,16β propionyloxy-17α prégnadiène-4,9(11) dione-3,20,
diméthyl-6α,16β dipropionyloxy-17α,21 prégnatriène-1,4,9(11) dione-3,20 et
chloro-21 diméthyl-6α,16β propionyloxy-17α prégnatriène-1,4,9(11) dione-3,20.

13. Epoxy-9,11β diméthyl-6α,16β dipropionyloxy-17α,21 prégnadiène-1,4 dione-3,20, époxy-9,11β diméthyl-6α,16β dipropionyloxy-17α,21 prégnène-4 dione-3,20,
acétoxy-21 époxy-9,11β diméthyl-6α,16β propionyloxy-17α prégnène-4 dione-3,20,
chloro-21 époxy-9,11β diméthyl-6α,16β propionyloxy-17α prégnène-4 dione-3,20 et
chloro-21 époxy-9,11β diméthyl-6α,16β propionyloxy-17α prégnadiène-1,4 dione-3,20.